# EUROPEAN PATENT SPECIFICATION

(11) **EP 4 085 373 B1**
(45) Date of publication and mention of the grant of the patent: **05.03.2025**
(21) Application number: 21702326.6
(22) Date of filing: 01.01.2021
(51) Int. Cl.: G02B 27/01, G06V 20/20, G06V 40/10, G06F 3/0346, G06T 7/73, G06F 3/03

(54) **JOINT INFRARED AND VISIBLE LIGHT VISUAL-INERTIAL OBJECT TRACKING**
KOMBINIERTE VISUELL-INERTIAL-OBJEKTVERFOLGUNG MITHILFE VON INFRAROT- UND SICHTBAREM LICHT
SUIVI COMBINÉ D'OBJETS VISUELS ET INERTIELS UTILISANT L'INFRAROUGE ET LA LUMIÈRE VISIBLE

(30) Priority: 03.01.2020 US 202016734172
(43) Date of publication of application: 09.11.2022
(73) Proprietor: META PLATFORMS TECHNOLOGIES, LLC, Menlo Park, CA 94025 (US)
(72) Inventor: FORSTER, Christian, Menlo Park, California 94025 (US); MELIM, Andrew, Menlo Park, California 94025 (US)
(74) Representative: Murgitroyd & Company
(86) International application number: PCT/US2021/012001
(87) International publication number: WO 2021/138637

(56) References cited:
- US-A1- 2019 325 274

## Description

### TECHNICAL FIELD

This disclosure generally relates to infrared-based object tracking, and more specifically methods, apparatus, and system for inertial-aided infrared and visible light tracking.

### BACKGROUND

Current AR/VR controllers are being tracked using the known patterns formed by infrared (IR) light emitting diodes (LEDs) on the controllers. Although each controller has an IMU and the IMU data could be used to determine the pose of the controller, the estimated pose will inevitably drift over time. Thus, periodically, the IMU-based pose estimations of the controller would need to be realigned with the observed patterns observed by the camera. In addition, tracking based on the IR LEDs have several shortcomings. For example, bright sunlight or other infrared light sources would cause tracking to fail. Furthermore, when the controller is close to the user's head, the IR LEDs may not be visible to allow for proper tracking.

Prior art document US 2019/325274 A1 discloses a method for tracking the poses of a wearable device, by recognizing feature points on the surface of the wearable device.

### SUMMARY OF PARTICULAR EMBODIMENTS

To address the foregoing problems, disclosed are methods, apparatuses, and a system, to track a controller by capturing a short exposure frame and a long exposure frame of an object alternately, such as performing an infrared (IR)-based tracking and a visual inertial odometry (VIO) tracking alternately by a camera. The present disclosure provides a method to realign a location of the controller by taking an IR image of the controller with a shorter exposure time and a visible-light image with a longer exposure time alternately. The method disclosed in the present application considers the condition of the environment to track the controller based on the IR-based observations or the visible-light observations. Furthermore, the method disclosed in the present application may re-initiate the tracking of the controller periodically or when the controller is visible in the field of view of the camera, so that an accuracy of the estimated pose of the controller can be improved over time.

The embodiments disclosed herein are only examples, and the scope of this disclosure is not limited to them. Particular embodiments may include all, some, or none of the components, elements, features, functions, operations, or steps of the embodiments disclosed herein. The method comprises, by a computing system, receiving motion data captured by one or more motion sensors of a wearable device. The method further comprises generating a pose of the wearable device based on the motion data. The method yet further comprises capturing a first frame of the wearable device by a camera using a first exposure time. The method additionally comprises identifying, in the first frame, a pattern of lights disposed on the wearable device. The method further comprises capturing a second frame of the wearable device by the camera using a second exposure time. The method further comprises identifying, in the second frame, predetermined features of the wearable device. In particular embodiments, the predetermined features may be features identified in a previous frame. The method yet further comprises adjusting the pose of the wearable device in an environment based on at least one of (1) the identified pattern of lights in the first frame and (2) the identified predetermined features in the second frame.

In accordance with one aspect of the present invention, there is provided a method comprising, by a computing system: receiving motion data captured by one or more motion sensors of a wearable device; generating a pose of the wearable device based on the motion data; capturing a first frame of the wearable device by a camera using a first exposure time; identifying, in the first frame, a pattern of lights disposed on the wearable device; capturing a second frame of the wearable device by the camera using a second exposure time; identifying, in the second frame, predetermined features of the wearable device; and adjusting the pose of the wearable device in an environment based on the identified pattern of lights in the first frame and the identified predetermined features in the second frame.

The camera captures the first frame of the wearable device using the first exposure time when the environment has a first light condition; and the second frame of the wearable device using the second exposure time when the environment has a second light condition.

In some embodiments, the second light condition may comprise one or more of: an environment having bright light; an environment having a light source to interfere the pattern of lights of the wearable device; and the camera not being able to capture the pattern of lights.

In some embodiments, the wearable device may be equipped with one or more inertial measurement units (IMUs) and one or more infrared (IR) light emitting diodes (LEDs); the first frame is an IR image; and the second frame is a visible-light image.

The second exposure time is longer than the first exposure time.

In some embodiments, the pose of the wearable device may be generated at a faster frequency than a frequency that the first frame and the second frame are captured.

In some embodiments, the method may further comprise: capturing a third frame of the wearable device by the camera using the second exposure time; identifying, in the third frame, one or more features corresponding to the predetermined features of the wearable device; determining correspondence data between the predetermined features and the one or more features; and tracking the wearable device in the environment based on the correspondence data.

The system comprises: the camera configured to capture the first frame and the second frame of the wearable device. Furthermore, the system is configured to identify the pattern of lights and the predetermined features of the wearable device; and to adjust the pose of the wearable device

In some embodiments, the camera may be located within a head-mounted device; and wherein the wearable device is a controller separated from the head-mounted device.

In some embodiments, the head-mounted device may comprise one or more processors, wherein the one or more processors are configured to implement the camera, the identifying unit, and the filter unit.

In accordance with a further aspect of the present invention, there is provided one or more computer-readable non-transitory storage media embodying software that is operable when executed to: receive motion data captured by one or more motion sensors of a wearable device; generate a pose of the wearable device based on the motion data; capture a first frame of the wearable device by a camera using a first exposure time; identify, in the first frame, a pattern of lights disposed on the wearable device; capture a second frame of the wearable device by the camera using a second exposure time; identify, in the second frame, predetermined features of the wearable device; and adjust the pose of the wearable device in an environment based on the identified pattern of lights in the first frame and the identified predetermined features in the second frame.

The software is further operable when executed to: capture the first frame of the wearable device using the first exposure time when the environment has a first light condition; and capture the second frame of the wearable device using the second exposure time when the environment has a second light condition.

In some embodiments, the second light condition may comprise one or more of: an environment having bright light; an environment having a light source to interfere the pattern of lights of the wearable device; and the camera not being able to capture the pattern of lights.

In some embodiments, the wearable device may be equipped with one or more inertial measurement units (IMUs) and one or more infrared (IR) light emitting diodes (LEDs); the first frame is an IR image; and the second frame is a visible-light image.

The second exposure time is longer than the first exposure time.

In some embodiments, the pose of wearable device may be generated at a faster frequency than a frequency that the first frame and the second frame are captured.

In some embodiments, the software may be further operable when executed to: capture a third frame of the wearable device by the camera using the second exposure time; identify, in the third frame, one or more features corresponding to the predetermined features of the wearable device; determine correspondence data between the predetermined features and the one or more features; and track the wearable device in the environment based on the adjusted pose and the correspondence data.

In some embodiments, the camera may be located within a head-mounted device; and wherein the wearable device is a remote controller separated from the head-mounted device.

In accordance with a further aspect of the present invention, there is provided a system comprising: one or more processors; and one or more computer-readable non-transitory storage media coupled to one or more of the processors and comprising instructions operable when executed by the one or more of the processors to cause the system to: receive motion data captured by one or more motion sensors of a wearable device; generate a pose of the wearable device based on the motion data; capture a first frame of the wearable device by a camera using a first exposure time; identify, in the first frame, a pattern of lights disposed on the wearable device; capture a second frame of the wearable device by the camera using a second exposure time; identify, in the second frame, predetermined features of the wearable device; and adjust the pose of the wearable device in an environment based on the identified pattern of lights in the first frame and the identified predetermined features in the second frame.

The instructions further cause the system to: capture the first frame of the wearable device using the first exposure time when the environment has a first light condition; and capture the second frame of the wearable device using the second exposure time when the environment has a second light condition.

The invention is defined by the attached claims directed to a method, a storage medium, and a system.

Certain aspects of the present disclosure and their embodiments may provide solutions to these or other challenges. There are, proposed herein, various embodiments which address one or more of the issues disclosed herein. The methods disclosed in the present disclosure may provide a tracking method for a controller, which adjusts the pose of the controller estimated by IMU data collected from the IMU(s) disposed on the controller based on an IR image and/or a visible-light image captured by a camera of the head-mounted device. The methods disclosed in the present disclosure may improve the accuracy of the pose of the controller, even if the user is under an environment with various light conditions or light interferences. Furthermore, particular embodiments disclosed in the present application may generate the pose of the controller based on the IMU data and the visible-light images, so that the IR-based tracking may be limited under a certain light condition to save power and potentially lower cost for manufacturing the controller. Therefore, the alternative tracking system disclosed in the present disclosure may improve the tracking task efficiently in various environment conditions.

Particular embodiments of the present disclosure may include or be implemented in conjunction with an artificial reality system. Artificial reality is a form of reality that has been adjusted in some manner before presentation to a user, which may include, e.g., a virtual reality (VR), an augmented reality (AR), a mixed reality (MR), a hybrid reality, or some combination and/or derivatives thereof. Artificial reality content may include completely generated content or generated content combined with captured content (e.g., real-world photographs). The artificial reality content may include video, audio, haptic feedback, or some combination thereof, and any of which may be presented in a single channel or in multiple channels (such as stereo video that produces a three-dimensional effect to the viewer). Additionally, in some embodiments, artificial reality may be associated with applications, products, accessories, services, or some combination thereof, that are, e.g., used to create content in an artificial reality and/or used in (e.g., perform activities in) an artificial reality. The artificial reality system that provides the artificial reality content may be implemented on various platforms, including a head-mounted display (HMD) connected to a host computer system, a standalone HMD, a mobile device or computing system, or any other hardware platform capable of providing artificial reality content to one or more viewers.

The embodiments disclosed herein are only examples, and the scope of this disclosure is not limited to them. Particular embodiments may include all, some, or none of the components, elements, features, functions, operations, or steps of the embodiments disclosed above. Embodiments according to the invention are in particular disclosed in the attached claims directed to a method, a storage medium, and a system.

### BRIEF DESCRIPTION OF THE DRAWINGS

The patent or application file contains drawings executed in color. Copies of this patent or patent application publication with color drawing(s) will be provided by the Office upon request and payment of the necessary fee.

The accompanying drawing figures incorporated in and forming a part of this specification illustrate several aspects of the disclosure, and together with the description serve to explain the principles of the disclosure.
**FIG. 1** illustrates an example diagram of a tracking system architecture.
**FIG. 2** illustrates an example embodiment of tracking a controller based on an IR images and/or a visible-light image.
**FIG. 3** illustrates an example embodiment of tracking the controller based on the identified pattern of lights and/or the identified features.
**FIG. 4** illustrates an example diagram of adjusting a pose of the controller.
**FIG. 5** illustrates an example diagram of locating the controller in a local or global map based on the adjusted pose of the controller.
**FIGS. 6A-6B** illustrate an embodiment of a method for adjusting a pose of the wearable device by capturing an IR image and a visible-light image alternately based on a first light condition in an environment.
**FIG. 7** illustrates an embodiment of a method for adjusting a pose of the wearable device by capturing a visible-light image based on a second light condition in an environment.
**FIG. 8** illustrates an example computer system.

### DESCRIPTION OF EXAMPLE EMBODIMENTS

For extensive services and functions provided by current AR/VR devices, a controller is commonly paired with the AR/VR devices to provide the user an easy, intuitive way to input instructions for the AR/VR devices. The controller is usually equipped with at least one inertial measurement units (IMUs) and infrared (IR) light emitting diodes (LEDs) for the AR/VR devices to estimate a pose of the controller and/or to track a location of the controller, such that the user may perform certain functions via the controller. For example, the user may use the controller to display a visual object in a corner of the room or generate a visual tag in an environment. The estimated pose of the controller will inevitably drift over time and require a realignment by an IR-based tracking. However, the IR-based tracking may be interfered by other LED light sources and/or under an environment having bright light. Furthermore, the IR-based tracking may fail due to the IR LEDs of the controller not being visible to allow for proper tracking. Particular embodiments disclosed in the present disclosure provide a method to alternately take an IR image and a visible-light image for adjusting the pose of the controller based on different light levels, environmental conditions, and/or a location of the controller.

Particular embodiments disclosed in the present disclosure provide a method to realign the pose of the controller utilizing an IR tracking or a feature tracking depending on whichever happens first. During an initialization of a controller, particular embodiments of the present application may predetermine certain features, e.g., reliable features to track the controller, by setting/painting on these features in a central module, so that the central module can identify these features in a visible-light image to adjust a pose of the controller when the pose of the controller drifts along operation.

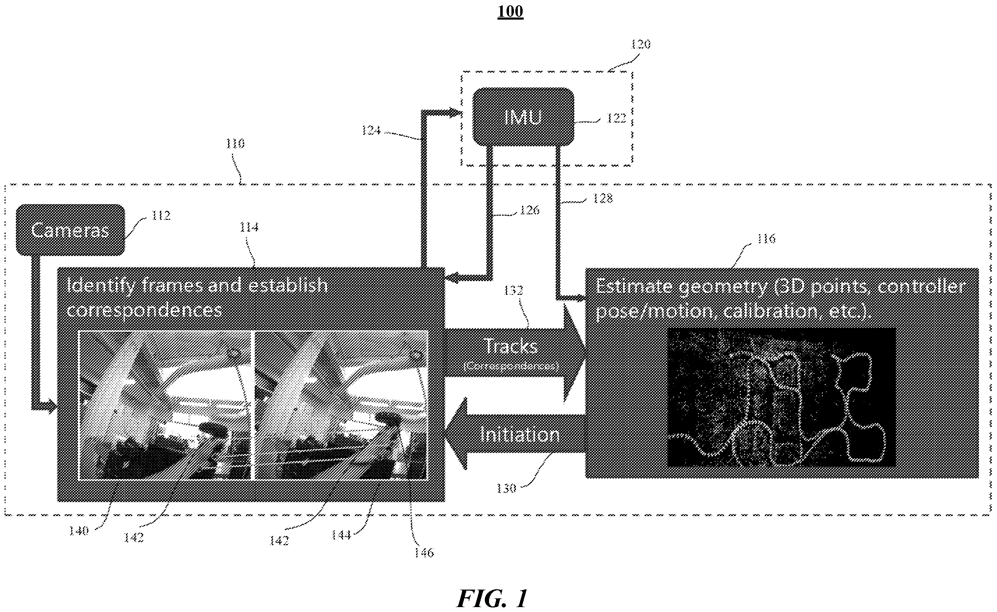

**FIG. 1** illustrates an example VIO-based SLAM tracking system architecture, in accordance with certain embodiments. The tracking system 100 comprises a central module 110 and at least one controller module 120. The central module 110 comprises a camera 112 configured to capture a frame of the controller module 120 in an environment, an identifying unit 114 configured to identify patches and features from the frame captured by the camera 112, and at least one processor 116 configured to estimate geometry of the central module 110 and the controller module 120. For example, the geometry comprises 3D points in a local map, a pose/motion of the controller module 120 and/or the central module 110, a calibration of the central module 110, and/or a calibration of the controller module 120. The controller module 120 comprises at least one IMU 122 configured to collect raw IMU data 128 of the controller module 120 upon receiving an instruction 124 from the central module 110, and to send the raw IMU data 128 to the processor 116 to generate a pose of the controller module 120, such that the central module 110 may learn and track a pose of the controller module 120 in the environment. The controller module 120 can also provide raw IMU data 126 to the identifying unit 114 for computing a prediction, such as correspondence data, for a corresponding module. Furthermore, the controller module 120 may comprise trackable markers selectively distributed on the controller module 120 to be tracked by the central module 110. For example, the trackable markers may be a plurality of light (e.g., light emitting diodes) or other trackable markers that can be tracked by the camera 112.

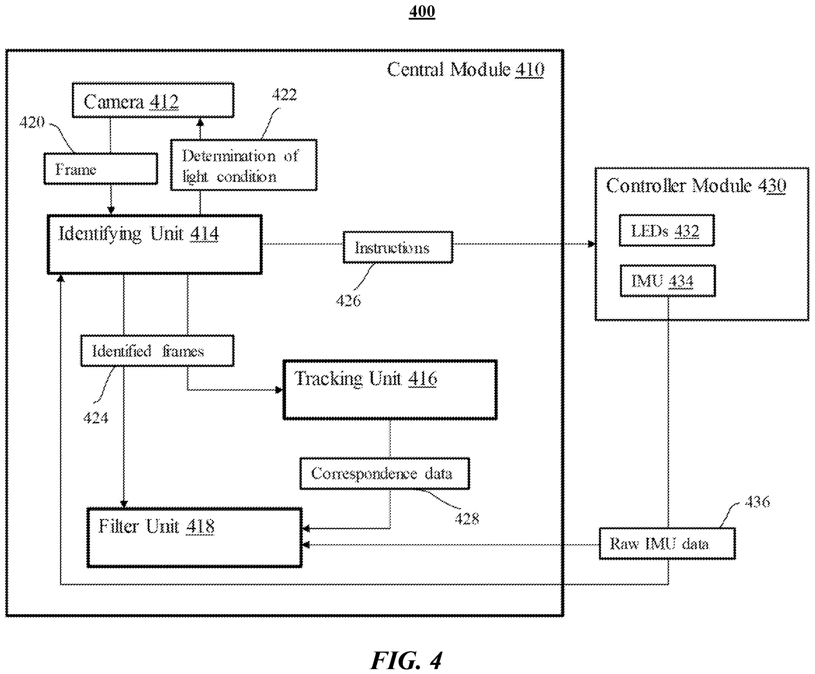

In particular embodiments, the identifying unit 114 of the central module 110 receives an instruction 130 to initiate the controller module 120. The identifying unit 114 instructs the camera 112 to capture a first frame of the controller module 120 for the initialization upon the receipt of the instruction 130. The first frame 140 may comprise one or more predetermined features 142 which are set or painted on in the central module 110. For example, the predetermined features 142 may be features identified in previous frames to track the controller module 120, and these identified features which are repeatedly recognized in the previous frames are considered reliable features for tracking the controller module 120. The camera 112 of the central module 110 may then start to capture a second frame 144 after the initialization of the controller module 120. For example, the processor 116 of the central module 110 may start to track the controller module 120 by capturing the second frame 144. In one embodiment, the second frame 144 may be a visible-light image which comprises the predetermined feature 142 of the controller module 120, so that the central module 110 may adjust the pose of the controller module 120 based on the predetermined feature 142 captured in the second frame 144. In another embodiment, the second frame may be an IR image which captures the plurality of lights disposed on the controller module 120, such that the central module 110 may realign the pose of the controller module 120 based on a pattern 146 of lights formed by the plurality of lights on the controller module 120. Also, the IR image can be used to track the controller module 120 based on the pattern 146 of lights, e.g., constellation of LEDs, disposed on the controller module 120, and furthermore, to update the processor 116 of the central module 110. In particular embodiments, the central module 110 may be set to take an IR image and a visible-light image alternately for realignment of the controller module 120. In particular embodiments, the central module 110 may determine to take either an IR image or a visible-light image for realignment of the controller module 120 based on a light condition of the environment. Detailed operations and actions performed at the central module 110 may be further described in FIG. 4.

In certain embodiments, the identifying unit 114 may further capture a third frame following the second frame 144 and identify, in the third frame, one or more patches corresponding to the predetermined feature 142. In this particular embodiment, the second frame 144 and the third frame, and potentially one or more next frames, are the visible-light frames, e.g., the frames taken with a long-exposure time, such that the central module 110 can track the controller module 120 based on the repeatedly-identified features over frames. The identifying unit 114 may then determine correspondence data 132 of a predetermined feature 142 between patches corresponding to each other identified in different frames, e.g., the second frame 144 and the third frame, and send the correspondence data 132 to the processor 116 for further analysis and service, such as adjusting the pose of the controller module 120 and generating state information of the controller module 120. In particular embodiments, the state information may comprise a pose, velocity, acceleration, spatial position and motion of the controller module 120, and potentially a previous route, of controller module 120 relative to an environment built by the series of frames captured by the cameras 112 of the central module 110.

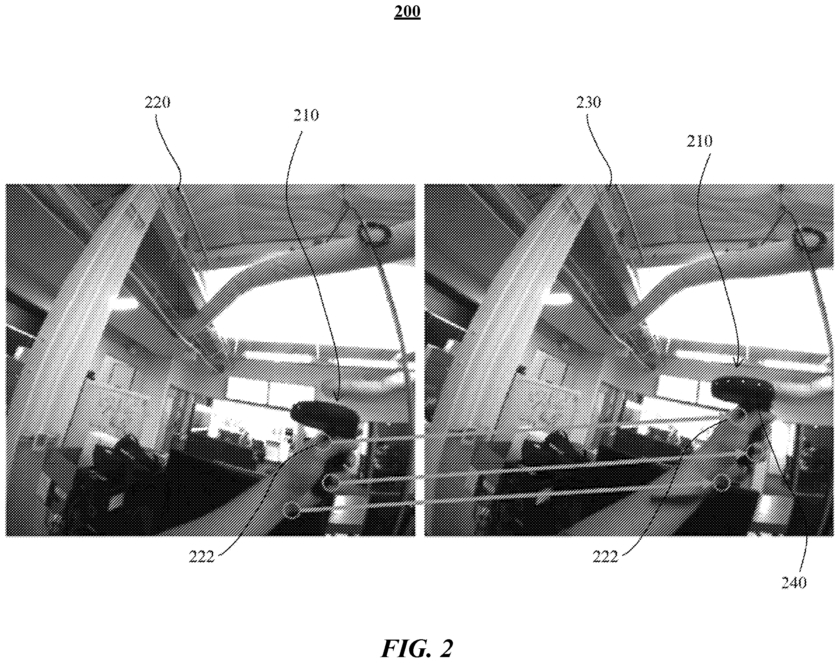

**FIG. 2** illustrates an example tracking system for a controller based on an IR image and/or a visible-light image, in accordance with certain embodiments. The tracking system 200 comprises a central module (not shown) and a controller module 210. The central module comprises a camera and at least one processor to track the controller module 210 in an environment. In particular embodiments, the camera of the central module may capture a first frame 220 to determine or set up predetermined features 222 of the controller module 210 for tracking during initialization stage. For example, during the initialization/startup phase of the controller module 210, a user would place the controller module 210 in a range of field of view (FOV) of the camera of the central module to initiate the controller module 210. The camera of the central module may capture the first frame 220 of the controller module 210 in this startup phase to determine one or more predetermined features 222 to track the controller module 210, such as an area where the purlicue of the hand overlaps with the controller module 120 and the ulnar border of the hand where represents a user's hand holding the controller module 120. In particular embodiments, the predetermined features 222 can also be painted on (e.g., via small QR codes). In particular embodiments, the predetermined feature 222 may be a corner of a table or any other trackable features identified in a visible-light frame. In particular embodiments, the predetermined feature 222 may be IR patterns "blobs" in an IR image, e.g., the constellations of LEDs captured in the IR image.

In particular embodiments, the controller module 210 comprises at least one IMU and a plurality of IR LEDs, such that the controller module 210 can be realigned during operation based on either a second frame 230 capturing a pattern 240 of the IR LEDs or a second frame 230 capturing the predetermined features 222. For example, the central module may generate a pose of the controller module 210 based on raw IMU data sending from the controller module 210. The generated pose of the controller module 210 may be shifted over time and required a realignment. The central module may determine to capture a second frame 230 which captures the controller module 210 for adjusting the generated pose of the controller 210 based on a light condition in the environment. In one embodiment, the second frame 230 may be an IR image comprising a pattern 240 of the IR LEDs. When the IR pattern is a known a priori, the second frame, which is an IR image, can be used to realign or track the controller module 210 without multiple frames. In another embodiment, the second frame 230 may be a visible-light image which is identified to comprise at least one predetermined feature 222. The visible-light image may be an RGB image, a CMYK image, or a greyscale image.

In particular embodiments, the central module may capture an IR image and a visible-light image alternately by a default setting, such that the central module may readjust the generated pose of the controller module 210 based on either the IR image or the visible-light image whichever is captured first for readjustment. In particular embodiments, the central module may capture the IR image when the environment comprises a first light condition. The first light condition may comprise one or more of an indoor environment, an environment not having bright light in the background, an environment not having a light source to interfere the pattern 240 of IR LEDs of the controller module 210. For example, the environment may not comprise other LEDs to interfere the pattern 240 formed by the IR LEDs of the central module to determine a location of the controller module 210.

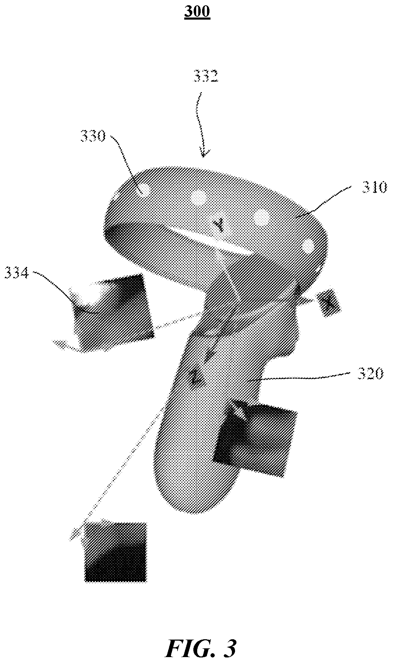

In particular embodiments, the central module may capture the visible image when the environment comprises a second light condition. The second light condition may comprise one or more of an environment having bright light, an environment having a light source to interfere the pattern 240 of IR LEDs of the controller module 210, and the camera of the central module not being able to capture the pattern of lights. For example, when a user is holding a controller implemented with the controller module 210 too close to a head-mounted device implemented with the central module, the camera of the central module cannot capture a complete pattern 240 formed by the IR LEDs of the controller module 210 to determine a location of the controller module 210 in the environment. Detailed operations and actions performed at the central module may be further described in FIGS. 3 to 7.

**FIG. 3** illustrates an example controller 300 implemented with a controller module, in accordance with certain embodiments. The controller 300 comprises a surrounding ring portion 310 and a handle portion 320. The controllers 300 is implemented with the controller module described in the present disclosure and includes a plurality of tracking features positioned in a corresponding tracking pattern. In particular embodiments, the tracking features can include, for example, fiducial markers or light emitting diodes (LED). In particular embodiments described herein the tracking features are LED lights, although other lights, reflectors, signal generators or other passive or active markers can be used in other embodiments. For example, the controller 300 may comprise a contrast feature on the ring portion 310 or the handle portion 320, e.g., a strip with contrast color around the surface of the ring portion 310, and/or a plurality of IR LEDs 330 embedded in the ring portion 310. The tracking features in the tracking patterns are configured to be accurately tracked by a tracking camera of a central module to determine a motion, orientation, and/or spatial position of the controller 300 for reproduction in a virtual/augmented environment. In particular embodiments, the controller 300 includes a constellation or pattern of lights 332 disposed on the ring portion 310.

In particular embodiment, the controller 300 comprises at least one predetermined feature 334 for the central module to readjust a pose of the controller 300. The pose of the controller 300 may be adjusted by a spatial movement (X-Y-Z positioning movement) determined based on the predetermined features 334 between frames. For example, the central module may determine an updated spatial position of the controller 300 in frame k+1, e.g., a frame captured during operation, and compare it with a previous spatial position of the controller 300 in frame k, e.g., a frame captured in the initialization of the controller 300, to readjust the pose of the controller 300.

**FIG. 4** illustrates an example diagram of a tracking system 400 comprising a central module 410 and a controller module 430, in accordance with certain embodiments. The central module 410 comprises a camera 412, an identifying unit 414, a tracking unit 416, and a filter unit 418 to perform a tracking/adjustment for the controller 420 in an environment. The controller module 430 comprises a plurality of LEDs 432 and at least one IMU 434. In particular embodiments, the identifying unit 414 of the central module 410 may send instructions 426 to initiate the controller module 430. In particular embodiments, the initialization for the controller module 430 may comprise capturing a first frame of the controller module 430 and predetermining one or more features in the first frame for tracking/identifying the controller module 430. The instructions 426 may indicate the controller module 430 to provide raw IMU data 436 for the central module 410 to track the controller module 430. The controller module 430 sends the raw IMU data 436 collected by the IMU 434 to the filter unit 418 of the central module 410 upon a receipt of the instructions 426, to order to generate/estimate a pose of the controller module 430 during operation. Furthermore, the controller module 430 sends the raw IMU data 436 to the identifying unit 414 for computing predictions of a corresponding module, e.g., correspondence data of the controller module 430. In particular embodiments, the central module 410 measures the pose of the controller module 430 at a frequency from 500Hz to 1 kHz.

After initialization of the controller module 430, the camera 412 of the central module 410 may capture a second frame when the controller module 430 is within a FOV range of the camera for a realignment of the generated pose of the controller module 430. In particular embodiments, the camera 412 may capture the second frame of the controller module 430 for realignment as an IR image or a visible-light image alternately by a default setting. For example, the camera 412 may capture an IR image and a visible-light image alternately at a slower frequency than the frequency of generating the pose of the controller module 430, e.g., 30 Hz, and utilize whichever image captured first or capable for realignment, such as an image capturing a trackable pattern of the LEDs 432 of the controller module 430 or an image capturing predetermined features for tracking the controller module 430.

In particular embodiments, the identifying unit 414 may determine a light condition in the environment to instruct the camera 412 to take a specific type of frame. For example, the camera 412 may provide the identifying unit 414 a frame 420 based on a determination of the light condition 422. In one embodiment, the camera 412 may capture an IR image comprising a pattern of LEDs 432 disposed on the controller module 430, when the environment does not have bright light in the background. In another embodiment, the camera 412 may capture a visible-light image of the controller module 430, when the environment has a similar light source to interfere the pattern of LEDs 432 of the controller module 430. In particular embodiments, the camera 412 captures an IR image using a first exposure time and captures a visible-light image using a second exposure time. The second exposure time may be longer than the first exposure time considering the movement of the user and/or the light condition of the environment.

In particular embodiments where no LEDs 432 of the controller module 430 are used, the central module 410 may track the controller module 430 based on visible-light images. A neural network may be used to find the controller module 430 in the visible-light images. The identifying unit 414 of the central module 410 may the identify features which are constantly observed over several frames, e.g., the predetermined features and/or reliable features for tracking the controller module 430, in the frames captured by the camera 412. The central module 410 may utilize these features to compute/adjust the pose of the controller module 430. In particular embodiments, the features may comprise patches of images corresponding to the controller module 430, such as the edges of the controller module 430.

In particular embodiments, the identifying unit 414 may further send the identified frames 424 to the filter unit 418 for adjusting the generated pose of the controller module 430. When the filter unit 418 receives an identified frame 418, which can either be an IR image capturing the pattern of lights or a visible-light image comprising patches for tracking the controller module 430, the filter unit 418 may determine a location of the controller module 430 in the environment based on the pattern of lights of the controller module 430 or the predetermined feature identified in the patches from the visible-light image. In particular embodiments, a patch may be a small image signature of a feature (e.g., corner or edge of the controller) that is distinct and easily identifiable in an image/frame, regardless of the angle at which the image was taken by the camera 412.

Furthermore, the filter unit 418 may also utilize these identified frames 424 to conduct extensive services and functions, such as generating a state of a user/device, locating the user/device locally or globally, and/or rendering a virtual tag/object in the environment. In particular embodiments, the filter unit 418 of the central module 410 may also use the raw IMU data 436 in assistance of generating the state of a user. In particular embodiments, the filter unit 418 may use the state information of the user relative to the controller module 430 in the environment based on the identified frames 424, to project a virtual object in the environment or set a virtual tag in a map via the controller module 430.

In particular embodiment, the identifying unit 414 may also send the identified frames 424 to the tracking unit 416 for tracking the controller module 430. The tracking unit 416 may determine correspondence data 428 based on the predetermined features in different identified frames 424, and track the controller module 430 based on the determined correspondence data 428.

In particular embodiments, the central module 410 captures at least the following frames to track/realign the controller module 430: (1) an IR image; (2) a visible-light image; (3) an IR image; and (4) a visible-light image. In a particular embodiment, the identifying unit 414 of the central module 410 may identify IR patterns in captured IR images. When the IR patterns in the IR images are matched against an a priori pattern, such as the constellation of LED positions on the controller module 430 identified in the first frame, a single IR image can be sufficient to be used by the filter unit 418 for state estimation and/or other computations. In another embodiment of a feature-based tracking, the identifying unit 414 of the central module 410 may identify a feature to track in a first visible-light image, and the identifying unit 414 may then try to identify the same feature in a second visible-light frame, which feature is corresponding to the feature identified in the first visible-light image. When the identifying unit 414 repeatedly observes the same feature over at least two visible-light frames, these observations, e.g., identified features, in these frames can be used by the filter unit 418 for state estimation and/or other computations. Furthermore, in particular embodiments, the central module 410 can also use a single visible-light frame to update the state estimation based on a three-dimensional model of the controller module 430, such as a computer-aided design (CAD) model of the controller module 430.

In particular embodiments, the tracking system 400 may be implemented in any suitable computing device, such as, for example, a personal computer, a laptop computer, a cellular telephone, a smartphone, a tablet computer, an augmented/virtual reality device, a head-mounted device, a portable smart device, a wearable smart device, or any suitable device which is compatible with the tracking system 400. In the present disclosure, a user which is being tracked and localized by the tracking device may be referred to a device mounted on a movable object, such as a vehicle, or a device attached to a person. In the present disclosure, a user may be an individual (human user), an entity (e.g., an enterprise, business, or third-party application), or a group (e.g., of individuals or entities) that interacts or communicates with the tracking system 400. In particular embodiments, the central module 410 may be implemented in a head-mounted device, and the controller module 430 may be implemented in a remote controller separated from the head-mounted device. The head-mounted device comprises one or more processors configured to implement the camera 412, the identifying unit 414, the tracking unit 416, and the filter unit 418 of the central module 410. In one embodiment, each of the processors is configured to implement the camera 412, the identifying unit 414, the tracking unit 416, and the filter unit 418 separately. The remote controller comprises one or more processors configured to implement the LEDs 432 and the IMU 434 of the controller module 430. In one embodiment, each of the processors is configured to implement the LEDs 432 and the IMU 434 separately.

This disclosure contemplates any suitable network to connect each element in the tracking system 400 or to connect the tracking system 400 with other systems. As an example and not by way of limitation, one or more portions of network may include an ad hoc network, an intranet, an extranet, a virtual private network (VPN), a local area network (LAN), a wireless LAN (WLAN), a wide area network (WAN), a wireless WAN (WWAN), a metropolitan area network (MAN), a portion of the Internet, a portion of the Public Switched Telephone Network (PSTN), a cellular telephone network, or a combination of two or more of these. Network may include one or more networks.

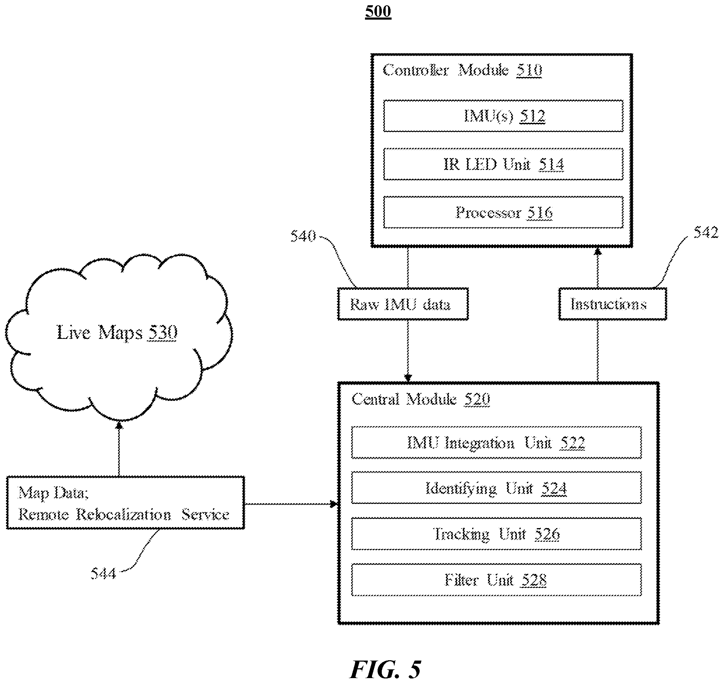

**FIG. 5** illustrates an example diagram of a tracking system 500 with mapping service, in accordance with certain embodiments. The tracking system 500 comprises a controller module 510, a central module 520, and a cloud 530. The controller module 510 comprises an IMU unit 512, a light unit 514, and a processor 516. The controller module 510 receives one or more instructions 542 from the central module 520 to perform specific functions. For example, the instruction 542 comprises, but is not limited to, an instruction to initiate the controller module 510, an instruction to switch off the light unit 514, and an instruction to tag a virtual object in the environment. The controller module 510 is configured to send raw IMU data 540 to the central module 420 for a pose estimation during operation, so that the processor 516 of the controller module 510 may perform the instructions 542 accurately in a map or in the environment.

The central module 520 comprises a camera 522, an identifying unit 524, a tracking unit 526, and a filter unit 528. The central module 520 may be configured to track the controller module 510 based on various methods, e.g., an estimated pose of the controller module 510 determined by the raw IMU data 540. Furthermore, the central module 520 may be configured to adjust the estimated pose of the controller module 510 during operation based on a frame of the controller module 510 captured by the camera 522. In particular embodiments, the identifying unit 524 of the central module 520 may determine a program to capture a frame of the controller module 510 based on a light condition of the environment. The program comprises, but is not limited to, capturing an IR image and a visible-light image alternately and capturing a visible-light image only. The IR image is captured by a first exposure time, and the visible-light image is captured by a second exposure time. In particular embodiments, the second exposure time may be longer than the first exposure time. The identifying unit 524 may then instruct the camera 522 to take a frame/image of the controller module 510 based on the determination, and the camera 522 would provide the identifying unit 524 a specific frame according to the determination. In particular embodiments, the identifying unit 524 may also instruct the controller module 510 to switch off the light unit 514 specific to a certain light condition, e.g., another LED source nearby, to save power.

The identifying unit 524 identifies the frame upon the receipt from the camera 522. In particular, the identifying unit 524 may receive a frame whichever is being captured first when the controller module 510 requires a readjustment of its pose. For example, the camera 522 captures an IR image and a visible-light image alternately at a slow rate, e.g., a frequency of 30 Hz, and then sends a frame to the identifying unit 524 when the controller module 510 is within the FOV of the camera 522. Therefore, the frame being captured could be either the IR image or the visible-light image. In particular embodiments, the identifying unit 524 may identify a pattern formed by the light unit 514 of the controller module 510 in the captured frame. The pattern formed by the light unit 514 may indicate that a position of the controller module 510 relative to the user/the central module 520 and/or the environment. For example, in response to a movement/rotation of the controller module 510, the pattern of the light unit 514 changes. In particular embodiments, the identifying unit 524 may identify predetermined features for tracking the controller module 510 in the captured frame. For example, the predetermined features of the controller module 510 may comprise a user's hand gesture when holding the controller module 510, so that the predetermined features may indicate a position of the controller module 510 relative to the user/the central module 520. The identifying unit 524 may then send the identified frames to the filter unit 528 for an adjustment of the pose of the controller module 528. In particular embodiments, the identifying unit 524 may also send the identified frames to the tracking unit 526 for tracking the controller unit 510.

The filter unit 528 generates a pose of the controller module 510 based on the received raw IMU data 540. In particular embodiments, the filter unit 528 generates the pose of the controller module 510 at a faster rate than a rate of capturing a frame of the controller module. For example, the filter unit 528 may estimate and update the pose of the controller module 510 at a rate of 500 Hz. The filter unit 528 then realign/readjust the pose of the controller module 510 based on the identified frames. In particular embodiments, the filter unit 528 may adjust the pose of the controller module 510 based on the pattern of the light unit 514 of the controller module 510 in the identified frame. In particular embodiments, the filter unit 528 may adjust the pose of the controller module 510 based on the predetermined features identified in the frame.

In particular embodiments, the tracking unit 526 may determine correspondence data based on the predetermined features identified in different frames. The correspondence data may comprise observations and measurements of the predetermined feature, such as a location of the predetermined feature of the controller module 510 in the environment. Furthermore, the tracking unit 526 may also perform a stereo computation collected near the predetermined feature to provide additional information for the central module 520 to track the controller module 510. In addition, the tracking unit 526 of the central module 520 may request a live map from the cloud 530 corresponding to the correspondence data. In particular embodiments, the live map may comprise map data 544. The tracking unit 526 of the central module 520 may also request a remote relocalization service 544 for the controller module 510 to be located in the live map locally or globally.

Furthermore, the filter unit 528 may estimate a state of the controller module 510 based on the correspondence data and the raw IMU data 540. In particular embodiments, the state of the controller module 510 may comprise a pose of the controller module 510 relative to an environment which is built based on the frames captured by the camera 522, e.g., a map built locally. In addition, the filter unit 528 may also send the state information of the controller module 510 to the cloud 530 for a global localization or an update of the map stored in the cloud 530 (e.g., with the environment built locally).

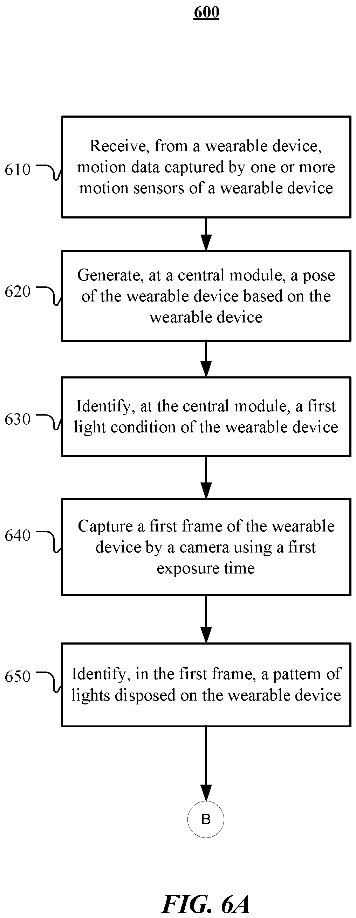

**FIG. 6A** illustrates an example method 600 for capturing an IR image based on a first light condition in an environment, in accordance with certain embodiments. A controller module of a tracking system may be implemented in the wearable device (e.g., a remote controller with input buttons, a smart puck with touchpad, etc.). A central module of the tracking system may be provided to or displayed on any computing system (e.g., an end user's device, such as a smartphone, virtual reality system, gaming system, etc.), and be paired with the controller module implemented in the wearable device. The method 600 may begin at step 610 receiving, from the wearable device, motion data captured by one or more motion sensors of the wearable device. In particular embodiments, the wearable device may be a controller. In particular embodiments, the wearable device may be equipped with one or more IMUs and one or more IR LEDs.

At step 620, the method 600 may generate, at the central module, a pose of the wearable device based on the motion data sent from the wearable device.

At step 630, the method 600 may identify, at the central module, a first light condition of the wearable device. In particular embodiments, the first light condition may comprise one or more of an indoor environment, an environment having dim light, an environment without a light source similar to the IR LEDs of the wearable device, and a camera of the central module being able to capture a pattern of IR LEDs of the wearable device for tracking.

At step 640, the method 600 may capture a first frame of the wearable device by a camera using a first exposure time. In particular embodiments, the first frame may be an IR image. In particular embodiments, the pose of the wearable device may be generated at a faster frequency than a frequency that the first frame is captured.

At step 650, the method 600 may identify, in the first frame, a pattern of lights disposed on the wearable device. In particular embodiments, the pattern of lights may be composed of the IR LEDs of the wearable device.

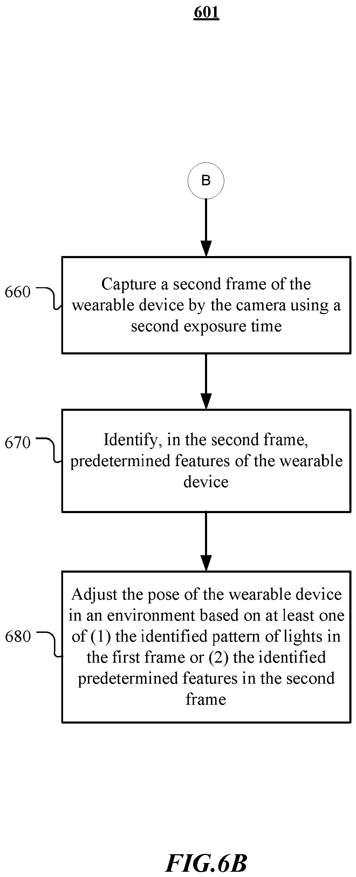

**FIG. 6B** illustrates an example method 601 for adjusting the pose of a wearable device by capturing the IR image and the visible-light image alternately based on the first light condition in the environment, in accordance with certain embodiments. The method 601 may begin, at step 660 follows the step 650 in the method 601, capturing a second frame of the wearable device by the camera using a second exposure time. In particular embodiments, the second exposure time may be longer than the first exposure time. In particular embodiments, the second frame may be a visible-light image. For example, the visible-light image may be an RGB image. In particular embodiments, the pose of the wearable device may be generated at a faster frequency than a frequency that the second frame is captured.

At step 670, the method 601 may identify, in the second frame, predetermined features of the wearable device. In particular embodiment, the predetermined features may be predetermined during the initialization/startup phase for the controller module. In particular embodiments, the predetermined features may be painted on (e.g., via small QR codes) in the controller module. In particular embodiments, the predetermined features may be reliable features for tracking the wearable device determined from previous operations. For example, the reliable feature may be a feature identified repeatedly in the previous frames for tracking the wearable device.

At step 680, the method 601 may adjust the pose of the wearable device in the environment based on at least one of (1) the identified pattern of lights in the first frame or (2) the identified predetermined features in the second frame. In particular embodiments, the method may adjust the pose of the wearable device based on the identified pattern of lights or the identified predetermined feature whichever is captured/identified first. In particular embodiments, the method may train or update neural networks based on the process of adjusting the pose of the wearable device. The trained neural networks may further be used in tracking and/or image refinement.

In particular embodiments, the method 601 may further capture a third frame of the wearable device by the camera using the second exposure time, identify, in the third frame, one or more features corresponding to the predetermined features of the wearable device, determine correspondence data between the predetermined features and the one or more features, and track the wearable device in the environment based on the correspondence data.

In particular embodiments, the computing system may comprise the camera configured to capture the first frame and the second frame of the wearable device, an identifying unit configured to identify the pattern of lights and the predetermined features of the wearable device, and a filter unit configured to adjust the pose of the wearable device. In particular embodiments, the central module may be located within a head-mounted device, and the controller module may be implemented in a controller separated from the head-mounted device. In particular embodiments, the head-mounted device may comprise one or more processors, and the one or more processors are configured to implement the camera, the identifying unit, and the filter unit.

In particular embodiments, the method 601 may be further configured to capture the first frame of the wearable device using the first exposure time when the environment has the first light condition. In particular embodiments, the method 601 may be further configured to capture the second frame of the wearable device using the second exposure time when the environment has a second light condition. The second light condition may comprise one or more of an environment having bright light, an environment having a light source to interfere the pattern of lights of the wearable device, and the camera not being able to capture the pattern of lights.

Particular embodiments may repeat one or more steps of the method of FIGS. 6A-6B, where appropriate. Although this disclosure describes and illustrates particular steps of the method of FIGS. 6A-6B as occurring in a particular order, this disclosure contemplates any suitable steps of the method of FIGS. 6A-6B occurring in any suitable order. Moreover, although this disclosure describes and illustrates an example method for local localization including the particular steps of the method of FIGS. 6A-6B, this disclosure contemplates any suitable method for local localization including any suitable steps, which may include all, some, or none of the steps of the method of FIGS. 6A-6B, where appropriate. Furthermore, although this disclosure describes and illustrates particular components, devices, or systems carrying out particular steps of the method of FIGS. 6A-6B, this disclosure contemplates any suitable combination of any suitable components, devices, or systems carrying out any suitable steps of the method of FIGS. 6A-6B.

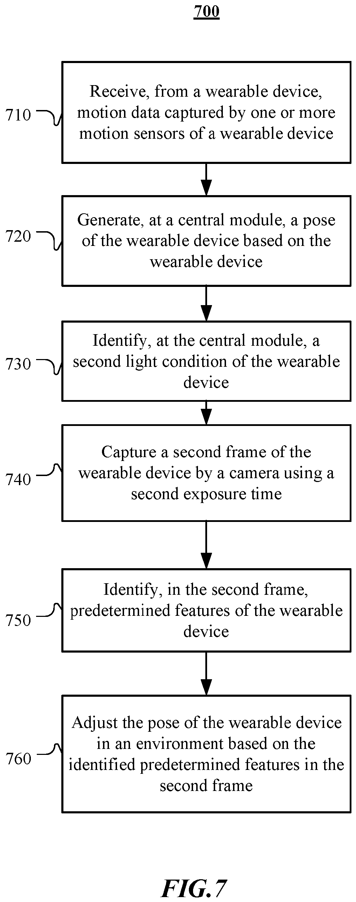

**FIG. 7** illustrates an example method 700 for adjusting a pose of the wearable device by capturing a visible-light image based on a second light condition in an environment, in accordance with certain embodiments. A controller module of a tracking system may be implemented in the wearable device (e.g., a remote controller with input buttons, a smart puck with touchpad, etc.). A central module of the tracking system may be provided to or displayed on any computing system (e.g., an end user's device, such as a smartphone, virtual reality system, gaming system, etc.), and be paired with the controller module implemented in the wearable device. The method 700 may begin at step 710 receiving, from the wearable device, motion data captured by one or more motion sensors of the wearable device. In particular embodiments, the wearable device may be a controller. In particular embodiments, the wearable device may be equipped with one or more IMUs and one or more IR LEDs.

At step 720, the method 700 may generate, at the central module, a pose of the wearable device based on the motion data sent from the wearable device.

At step 730, the method 700 may identify, at the central module, a second light condition of the wearable device. In particular embodiments, the second light condition may comprise one or more of an environment having bright light, an environment having a light source similar to the IR LEDs of the wearable device, and the camera not being able to capture the pattern of lights.

At step 740, the method 700 may capture a second frame of the wearable device by the camera using a second exposure time. In particular embodiments, the second frame may be a visible-light image. For example, the visible-light image may be an RGB image. In particular embodiments, the pose of the wearable device may be generated at a faster frequency than a frequency that the second frame is captured.

At step 750, the method 700 may identify, in the second frame, predetermined features of the wearable device. In particular embodiment, the predetermined features may be predetermined during the initialization/startup phase for the controller module. In particular embodiments, the predetermined features may be painted on (e.g., via small QR codes) in the controller module. In particular embodiments, the predetermined features may be reliable features for tracking the wearable device determined from previous operations. For example, the reliable feature may be a feature identified repeatedly in the previous frames for tracking the wearable device.

At step 760, the method 700 may adjust the pose of the wearable device in the environment based on the identified predetermined features in the second frame.

In particular embodiments, the method 700 may further capture a third frame of the wearable device by the camera using the second exposure time, identify, in the third frame, one or more features corresponding to the predetermined features of the wearable device, determine correspondence data between the predetermined features and the one or more features, and track the wearable device in the environment based on the correspondence data.

In particular embodiments, the computing system may comprise the camera configured to capture the first frame and the second frame of the wearable device, an identifying unit configured to identify the pattern of lights and the predetermined features of the wearable device, and a filter unit configured to adjust the pose of the wearable device. In particular embodiments, the central module may be located within a head-mounted device, and the controller module may be implemented in a controller separated from the head-mounted device. In particular embodiments, the head-mounted device may comprise one or more processors, and the one or more processors are configured to implement the camera, the identifying unit, and a filter unit.

In particular embodiments, the method 700 may be further configured to capture the second frame of the wearable device using the second exposure time when the environment has a second light condition. The second light condition may comprise one or more of an environment having bright light, an environment having a light source to interfere the pattern of lights of the wearable device, and the camera not being able to capture the pattern of lights.

Particular embodiments may repeat one or more steps of the method of FIG. 7, where appropriate. Although this disclosure describes and illustrates particular steps of the method of FIG. 7 as occurring in a particular order, this disclosure contemplates any suitable steps of the method of FIG. 7 occurring in any suitable order.

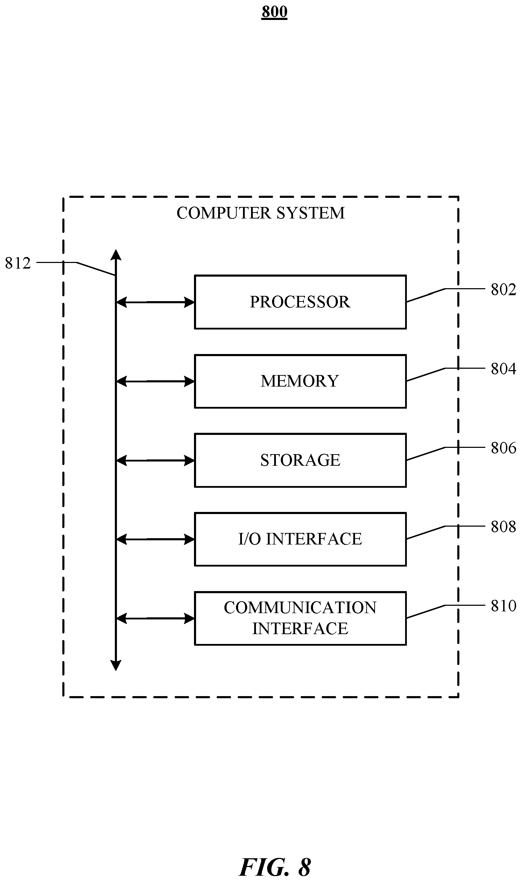

**FIG. 8** illustrates an example computer system 800. In particular embodiments, one or more computer systems 800 perform one or more steps of one or more methods described or illustrated herein. In particular embodiments, one or more computer systems 800 provide functionality described or illustrated herein. In particular embodiments, software running on one or more computer systems 800 performs one or more steps of one or more methods described or illustrated herein or provides functionality described or illustrated herein. Particular embodiments include one or more portions of one or more computer systems 800. Herein, reference to a computer system may encompass a computing device, and vice versa, where appropriate. Moreover, reference to a computer system may encompass one or more computer systems, where appropriate.

This disclosure contemplates any suitable number of computer systems 800. This disclosure contemplates computer system 800 taking any suitable physical form. As example and not by way of limitation, computer system 800 may be an embedded computer system, a system-on-chip (SOC), a single-board computer system (SBC) (such as, for example, a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, a tablet computer system, an augmented/virtual reality device, or a combination of two or more of these. Where appropriate, computer system 800 may include one or more computer systems 800; be unitary or distributed; span multiple locations; span multiple machines; span multiple data centers; or reside in a cloud, which may include one or more cloud components in one or more networks. Where appropriate, one or more computer systems 800 may perform without substantial spatial or temporal limitation one or more steps of one or more methods described or illustrated herein. As an example and not by way of limitation, one or more computer systems 800 may perform in real time or in batch mode one or more steps of one or more methods described or illustrated herein. One or more computer systems 800 may perform at different times or at different locations one or more steps of one or more methods described or illustrated herein, where appropriate.

In particular embodiments, computer system 800 includes a processor 802, memory 804, storage 806, an input/output (I/O) interface 808, a communication interface 810, and a bus 812. Although this disclosure describes and illustrates a particular computer system having a particular number of particular components in a particular arrangement, this disclosure contemplates any suitable computer system having any suitable number of any suitable components in any suitable arrangement.

In particular embodiments, processor 802 includes hardware for executing instructions, such as those making up a computer program. As an example and not by way of limitation, to execute instructions, processor 802 may retrieve (or fetch) the instructions from an internal register, an internal cache, memory 804, or storage 806; decode and execute them; and then write one or more results to an internal register, an internal cache, memory 804, or storage 806. In particular embodiments, processor 802 may include one or more internal caches for data, instructions, or addresses. This disclosure contemplates processor 802 including any suitable number of any suitable internal caches, where appropriate. As an example and not by way of limitation, processor 802 may include one or more instruction caches, one or more data caches, and one or more translation lookaside buffers (TLBs). Instructions in the instruction caches may be copies of instructions in memory 804 or storage 806, and the instruction caches may speed up retrieval of those instructions by processor 802. Data in the data caches may be copies of data in memory 804 or storage 806 for instructions executing at processor 802 to operate on; the results of previous instructions executed at processor 802 for access by subsequent instructions executing at processor 802 or for writing to memory 804 or storage 806; or other suitable data. The data caches may speed up read or write operations by processor 802. The TLBs may speed up virtual-address translation for processor 802. In particular embodiments, processor 802 may include one or more internal registers for data, instructions, or addresses. This disclosure contemplates processor 802 including any suitable number of any suitable internal registers, where appropriate. Where appropriate, processor 802 may include one or more arithmetic logic units (ALUs); be a multicore processor; or include one or more processors 802. Although this disclosure describes and illustrates a particular processor, this disclosure contemplates any suitable processor.

In particular embodiments, memory 804 includes main memory for storing instructions for processor 802 to execute or data for processor 802 to operate on. As an example and not by way of limitation, computer system 800 may load instructions from storage 806 or another source (such as, for example, another computer system 800) to memory 804. Processor 802 may then load the instructions from memory 804 to an internal register or internal cache. To execute the instructions, processor 802 may retrieve the instructions from the internal register or internal cache and decode them. During or after execution of the instructions, processor 802 may write one or more results (which may be intermediate or final results) to the internal register or internal cache. Processor 802 may then write one or more of those results to memory 804. In particular embodiments, processor 802 executes only instructions in one or more internal registers or internal caches or in memory 804 (as opposed to storage 806 or elsewhere) and operates only on data in one or more internal registers or internal caches or in memory 804 (as opposed to storage 806 or elsewhere). One or more memory buses (which may each include an address bus and a data bus) may couple processor 802 to memory 804. Bus 812 may include one or more memory buses, as described below. In particular embodiments, one or more memory management units (MMUs) reside between processor 802 and memory 804 and facilitate accesses to memory 804 requested by processor 802. In particular embodiments, memory 804 includes random access memory (RAM). This RAM may be volatile memory, where appropriate. Where appropriate, this RAM may be dynamic RAM (DRAM) or static RAM (SRAM). Moreover, where appropriate, this RAM may be single-ported or multi-ported RAM. This disclosure contemplates any suitable RAM. Memory 804 may include one or more memories 804, where appropriate. Although this disclosure describes and illustrates particular memory, this disclosure contemplates any suitable memory.

In particular embodiments, storage 806 includes mass storage for data or instructions. As an example and not by way of limitation, storage 806 may include a hard disk drive (HDD), a floppy disk drive, flash memory, an optical disc, a magneto-optical disc, magnetic tape, or a Universal Serial Bus (USB) drive or a combination of two or more of these. Storage 806 may include removable or non-removable (or fixed) media, where appropriate. Storage 806 may be internal or external to computer system 800, where appropriate. In particular embodiments, storage 806 is non-volatile, solid-state memory. In particular embodiments, storage 806 includes read-only memory (ROM). Where appropriate, this ROM may be mask-programmed ROM, programmable ROM (PROM), erasable PROM (EPROM), electrically erasable PROM (EEPROM), electrically alterable ROM (EAROM), or flash memory or a combination of two or more of these. This disclosure contemplates mass storage 806 taking any suitable physical form. Storage 806 may include one or more storage control units facilitating communication between processor 802 and storage 806, where appropriate. Where appropriate, storage 806 may include one or more storages 806. Although this disclosure describes and illustrates particular storage, this disclosure contemplates any suitable storage.

In particular embodiments, I/O interface 808 includes hardware, software, or both, providing one or more interfaces for communication between computer system 800 and one or more I/O devices. Computer system 800 may include one or more of these I/O devices, where appropriate. One or more of these I/O devices may enable communication between a person and computer system 800. As an example and not by way of limitation, an I/O device may include a keyboard, keypad, microphone, monitor, mouse, printer, scanner, speaker, still camera, stylus, tablet, touch screen, trackball, video camera, another suitable I/O device or a combination of two or more of these. An I/O device may include one or more sensors. This disclosure contemplates any suitable I/O devices and any suitable I/O interfaces 808 for them. Where appropriate, I/O interface 808 may include one or more device or software drivers enabling processor 802 to drive one or more of these I/O devices. I/O interface 808 may include one or more I/O interfaces 808, where appropriate. Although this disclosure describes and illustrates a particular I/O interface, this disclosure contemplates any suitable I/O interface.

In particular embodiments, communication interface 810 includes hardware, software, or both providing one or more interfaces for communication (such as, for example, packet-based communication) between computer system 800 and one or more other computer systems 800 or one or more networks. As an example and not by way of limitation, communication interface 810 may include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 810 for it. As an example and not by way of limitation, computer system 800 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, computer system 800 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination of two or more of these. Computer system 800 may include any suitable communication interface 810 for any of these networks, where appropriate. Communication interface 810 may include one or more communication interfaces 810, where appropriate. Although this disclosure describes and illustrates a particular communication interface, this disclosure contemplates any suitable communication interface.

In particular embodiments, bus 812 includes hardware, software, or both coupling components of computer system 800 to each other. As an example and not by way of limitation, bus 812 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination of two or more of these. Bus 812 may include one or more buses 812, where appropriate. Although this disclosure describes and illustrates a particular bus, this disclosure contemplates any suitable bus or interconnect.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

Herein, "or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

According to various embodiments, an advantage of features herein is that a pose of a controller associated with a central module in a tracking system can be efficiently realigned during operation. The central module can realign the controller based on either an IR constellation tracking or a VIO-based tracking, such that the central module may track the controller in real-time and accurately without any restrictions from the environment. Particular embodiments of the present disclosure also enable to track the controller when LEDs disposed on the controller fail. Furthermore, when the central module determines that the IR constellation tracking is compromised, the central module can switch off the LEDs on the controller for power saving. Therefore, particular embodiments disclosed in the present disclosure may provide an improved, power-efficient tracking method for the controller.

While processes in the figures may show a particular order of operations performed by certain embodiments of the invention, it should be understood that such order is exemplary (e.g., alternative embodiments may perform the operations in a different order, combine certain operations, overlap certain operations, etc.).

While the invention has been described in terms of several embodiments, those skilled in the art will recognize that the invention is not limited to the embodiments described, but is defined by the appended claims.

## Claims

1. A method (600, 601) comprising, by a computing system (800):
receiving (610) motion data captured by one or more motion sensors of a wearable device;
generating (620) a pose of the wearable device based on the motion data;
determining (630) that an environment of the device has a first light condition;
responsive to determining the first light condition, capturing (640) a first frame of the wearable device by a camera (112, 412, 522) using a first exposure time;
identifying (650), in the first frame, a pattern of lights disposed on the wearable device;
determining that an environment of the device has a second light condition;
responsive to determining the second light condition, capturing (660) a second frame of the wearable device by the camera (112, 412, 522) using a second exposure time that is longer than the first exposure time;
identifying (670), in the second frame, predetermined features of the wearable device; and
adjusting (680) the pose of the wearable device in an environment based on (1) the identified pattern of lights in the first frame and (2) the identified predetermined features in the second frame.

2. The method of claim 1, wherein the second light condition comprises one or more of:
an environment having bright light;
an environment having a light source to interfere the pattern of lights of the wearable device; and
the camera (112, 412, 522) not being able to capture the pattern of lights.

3. The method of claim 1 or claim 2, wherein:
the wearable device is equipped with one or more inertial measurement units IMUs (122, 434)and one or more infrared, IR, light emitting diodes, LEDs, (240, 330, 432);
the first frame is an IR image; and
the second frame is a visible-light image.

4. The method of any one of claims 1 to 3, wherein the pose of the wearable device is generated at a faster frequency than a frequency that the first frame and the second frame are captured.

5. The method of any one of the preceding claims, further comprising:
capturing a third frame of the wearable device by the camera (112, 412, 522) using the second exposure time;
identifying, in the third frame, one or more features corresponding to the predetermined features of the wearable device;
determining correspondence data between the predetermined features and the one or more features; and
tracking the wearable device in the environment based on the correspondence data.

6. The method of any one of the preceding claims, wherein the computing system (800) comprises:
the camera (112, 412, 522) configured to capture the first frame and the second frame of the wearable device;
an identifying unit configured to identify the pattern of lights and the predetermined features of the wearable device; and
a filter unit configured to adjust the pose of the wearable device.

7. The method of any one of the preceding claims,
wherein the camera (112, 412, 522) is located within a head-mounted device; and
wherein the wearable device is a controller separated from the head-mounted device; and preferably wherein the head-mounted device comprises one or more processors, wherein the one or more processors are configured to implement the camera (112, 412, 522), the identifying unit, and the filter unit.

8. One or more computer-readable non-transitory storage media embodying software that is operable when executed to:
receive (610) motion data captured by one or more motion sensors of a wearable device;
generate (620) a pose of the wearable device based on the motion data;
determine (630) that an environment of the device has a first light condition;
responsive to determining the first light condition, capture (640) a first frame of the wearable device by a camera (112, 412, 522) using a first exposure time;
identify (650), in the first frame, a pattern of lights disposed on the wearable device;
determine that an environment of the device has a second light condition;
responsive to determining the second light condition, capture (660) a second frame of the wearable device by the camera (112, 412, 522) using a second exposure time that is longer than the first exposure time;
identify (670), in the second frame, predetermined features of the wearable device; and
adjust (680) the pose of the wearable device in an environment based on (1) the identified pattern of lights in the first frame and (2) the identified predetermined features in the second frame.

9. The media of claim 8, wherein the second light condition comprises one or more of:
an environment having bright light;
an environment having a light source to interfere the pattern of lights of the wearable device; and
the camera (112, 412, 522) not being able to capture the pattern of lights.

10. The media of claim 8 or claim 9, wherein:
the wearable device is equipped with one or more inertial measurement units, IMUs (122, 434)and one or more infrared, IR, light emitting diodes, LEDs (240, 330, 432);
the first frame is an IR image; and
the second frame is a visible-light image;

11. The media of claim 8, claim 9 or claim 10 wherein the pose of wearable device is generated at a faster frequency than a frequency that the first frame and the second frame are captured.

12. The media of any one of claims 8 to 11, wherein the software is further operable when executed to:
capture a third frame of the wearable device by the camera (112, 412, 522) using the second exposure time;
identify, in the third frame, one or more features corresponding to the predetermined features of the wearable device;
determine correspondence data between the predetermined features and the one or more features; and
track the wearable device in the environment based on the adjusted pose and the correspondence data.

13. The media of any one of claims 8 to 12,
wherein the camera (112, 412, 522) is located within a head-mounted device; and
wherein the wearable device is a remote controller separated from the head-mounted device.

14. A system comprising: one or more processors (802); and one or more computer-readable non-transitory storage media (806)coupled to one or more of the processors and comprising instructions operable when executed by the one or more of the processors to cause the system to:
receive (610) motion data captured by one or more motion sensors of a wearable device;
generate (620) a pose of the wearable device based on the motion data;
determine (630) that an environment of the device has a first light condition;
responsive to determining the first light condition, capture (640) a first frame of the wearable device by a camera (112, 412, 522) using a first exposure time;
identify (650), in the first frame, a pattern of lights disposed on the wearable device;
determine that an environment of the device has a second light condition;
responsive to determining the second light condition, capture (660) a second frame of the wearable device by the camera (112, 412, 522) using a second exposure time that is longer than the first exposure time;
identify (670), in the second frame, predetermined features of the wearable device; and
adjust (680) the pose of the wearable device in an environment based on (1) the identified pattern of lights in the first frame and (2) the identified predetermined features in the second frame.

## Patentansprüche

1. Ein Verfahren (600, 601), das, durch ein Rechensystem (800), Folgendes beinhaltet:
Empfangen (610) von Bewegungsdaten, die von einem oder mehreren Bewegungssensoren einer tragbaren Vorrichtung erfasst werden;
Erzeugen (620) einer Pose der tragbaren Vorrichtung basierend auf den Bewegungsdaten;
Bestimmen (630), dass eine Umgebung der Vorrichtung eine erste Lichtbedingung aufweist;
als Reaktion auf das Bestimmen der ersten Lichtbedingung, Erfassen (640) eines ersten Frames der tragbaren Vorrichtung durch eine Kamera (112, 412, 522) unter Verwendung einer ersten Belichtungszeit;
Identifizieren (650), in dem ersten Frame, eines Musters von Lichtern, die auf der tragbaren Vorrichtung angeordnet sind;
Bestimmen, dass eine Umgebung der Vorrichtung eine zweite Lichtbedingung aufweist; als Reaktion auf das Bestimmen der zweiten Lichtbedingung, Erfassen (660) eines zweiten Frames der tragbaren Vorrichtung durch die Kamera (112, 412, 522) unter Verwendung einer zweiten Belichtungszeit, die länger als die erste Belichtungszeit ist;
Identifizieren (670), in dem zweiten Frame, vorbestimmter Merkmale der tragbaren Vorrichtung; und
Anpassen (680) der Pose der tragbaren Vorrichtung in einer Umgebung basierend auf (1) dem identifizierten Muster von Lichtern in dem ersten Frame und (2) den identifizierten vorbestimmten Merkmalen in dem zweiten Frame.

2. Verfahren gemäß Anspruch 1, wobei die zweite Lichtbedingung eines oder mehrere von Folgendem beinhaltet:
eine Umgebung mit hellem Licht;
eine Umgebung mit einer Lichtquelle, die das Muster von Lichtern der tragbaren Vorrichtung stört; und
die Kamera (112, 412, 522), die nicht in der Lage ist, das Muster von Lichtern zu erfassen.

3. Verfahren gemäß Anspruch 1 oder Anspruch 2, wobei:
die tragbare Vorrichtung mit einer oder mehreren Trägheitsmesseinheiten, IMUs, (122, 434) und einer oder mehreren Infrarot-, IR-, Leuchtdioden, LEDs, (240, 330, 432) ausgestattet ist;
das erste Frame ein IR-Bild ist; und
das zweite Frame ein Bild mit sichtbarem Licht ist.

4. Verfahren gemäß einem der Ansprüche 1 bis 3, wobei die Pose der tragbaren Vorrichtung mit einer schnelleren Frequenz erzeugt wird als eine Frequenz, mit der das erste Frame und das zweite Frame erfasst werden.

5. Verfahren gemäß einem der vorhergehenden Ansprüche, das ferner Folgendes beinhaltet:
Erfassen eines dritten Frames der tragbaren Vorrichtung durch die Kamera (112, 412, 522) unter Verwendung der zweiten Belichtungszeit;
Identifizieren, in dem dritten Frame, eines oder mehrerer Merkmale, die den vorbestimmten Merkmalen der tragbaren Vorrichtung entsprechen;
Bestimmen von Entsprechungsdaten zwischen den vorbestimmten Merkmalen und dem einen oder den mehreren Merkmalen; und
Verfolgen der tragbaren Vorrichtung in der Umgebung basierend auf den Korrespondenzdaten.

6. Verfahren gemäß einem der vorhergehenden Ansprüche, wobei das Rechensystem (800) Folgendes beinhaltet:
die Kamera (112, 412, 522), die so konfiguriert ist, dass sie das erste Frame und das zweite Frame der tragbaren Vorrichtung erfasst;
eine Identifizierungseinheit, die so konfiguriert ist, dass sie das Muster von Lichtern und die vorbestimmten Merkmale der tragbaren Vorrichtung identifiziert; und
eine Filtereinheit, die so konfiguriert ist, dass sie die Pose der tragbaren Vorrichtung anpasst.

7. Verfahren gemäß einem der vorhergehenden Ansprüche,
wobei die Kamera (112, 412, 522) innerhalb einer an einem Kopf montierten Vorrichtung befindlich ist; und
wobei die tragbare Vorrichtung eine von der an dem Kopf montierten Vorrichtung getrennte Steuereinheit ist; und vorzugsweise wobei die an dem Kopf montierte Vorrichtung einen oder mehrere Prozessoren beinhaltet, wobei der eine oder die mehreren Prozessoren so konfiguriert sind, dass sie die Kamera (112, 412, 522), die Identifizierungseinheit und die Filtereinheit implementieren.

8. Ein oder mehrere computerlesbare, nicht transitorische Speichermedien, die Software verkörpern, die bei Ausführung für Folgendes betreibbar ist:
Empfangen (610) von Bewegungsdaten, die von einem oder mehreren Bewegungssensoren einer tragbaren Vorrichtung erfasst werden;
Erzeugen (620) einer Pose der tragbaren Vorrichtung basierend auf den Bewegungsdaten;
Bestimmen (630), dass eine Umgebung der Vorrichtung eine erste Lichtbedingung aufweist;
als Reaktion auf das Bestimmen der ersten Lichtbedingung, Erfassen (640) eines ersten Frames der tragbaren Vorrichtung durch eine Kamera (112, 412, 522) unter Verwendung einer ersten Belichtungszeit;
Identifizieren (650), in dem ersten Frame, eines Musters von Lichtern, die auf der tragbaren Vorrichtung angeordnet sind;
Bestimmen, dass eine Umgebung der Vorrichtung eine zweite Lichtbedingung aufweist; als Reaktion auf das Bestimmen der zweiten Lichtbedingung, Erfassen (660) eines zweiten Frames der tragbaren Vorrichtung durch die Kamera (112, 412, 522) unter Verwendung einer zweiten Belichtungszeit, die länger als die erste Belichtungszeit ist;
Identifizieren (670), in dem zweiten Frame, von vorbestimmten Merkmalen der tragbaren Vorrichtung; und
Anpassen (680) der Pose der tragbaren Vorrichtung in einer Umgebung basierend auf (1) dem identifizierten Muster von Lichtern in dem ersten Frame und (2) den identifizierten vorbestimmten Merkmalen in dem zweiten Frame.

9. Medien gemäß Anspruch 8, wobei die zweite Lichtbedingung eines oder mehrere von Folgendem beinhaltet:
eine Umgebung mit hellem Licht;
eine Umgebung mit einer Lichtquelle, die das Muster von Lichtern der tragbaren Vorrichtung stört; und
die Kamera (112, 412, 522), die nicht in der Lage ist, das Muster von Lichtern zu erfassen.

10. Medien gemäß Anspruch 8 oder Anspruch 9, wobei:
die tragbare Vorrichtung mit einer oder mehreren Trägheitsmesseinheiten, IMUs, (122, 434) und einer oder mehreren Infrarot-, IR-, Leuchtdioden, LEDs, (240, 330, 432) ausgestattet ist;
das erste Frame ein IR-Bild ist; und
das zweite Frame ein Bild mit sichtbarem Licht ist.

11. Medien gemäß Anspruch 8, Anspruch 9 oder Anspruch 10, wobei die Pose der tragbaren Vorrichtung mit einer schnelleren Frequenz erzeugt wird als eine Frequenz, mit der das erste Frame und das zweite Frame erfasst werden.

12. Medien gemäß einem der Ansprüche 8 bis 11, wobei die Software bei Ausführung ferner für Folgendes betreibbar ist:
Erfassen eines dritten Frames der tragbaren Vorrichtung durch die Kamera (112, 412, 522) unter Verwendung der zweiten Belichtungszeit;
Identifizieren, in dem dritten Frame, eines oder mehrerer Merkmale, die den vorbestimmten Merkmalen der tragbaren Vorrichtung entsprechen;
Bestimmen von Korrespondenzdaten zwischen den vorbestimmten Merkmalen und dem einen oder den mehreren Merkmalen; und
Verfolgen der tragbaren Vorrichtung in der Umgebung basierend auf der angepassten Pose und den Korrespondenzdaten.

13. Medien gemäß einem der Ansprüche 8 bis 12,
wobei die Kamera (112, 412, 522) innerhalb einer an dem Kopf montierten Vorrichtung befindlich ist; und
wobei die tragbare Vorrichtung eine von der an dem Kopf montierten Vorrichtung getrennte entfernte Steuereinheit ist.

14. Ein System, das Folgendes beinhaltet: einen oder mehrere Prozessoren (802); und ein oder mehrere computerlesbare, nicht transitorische Speichermedien (806), die mit einem oder mehreren der Prozessoren gekoppelt sind und Befehle beinhalten, die, wenn sie von dem einen oder den mehreren Prozessoren ausgeführt werden, betreibbar sind, um das System zu Folgendem veranlassen:
Empfangen (610) von Bewegungsdaten, die von einem oder mehreren Bewegungssensoren einer tragbaren Vorrichtung erfasst werden;
Erzeugen (620) einer Pose der tragbaren Vorrichtung basierend auf den Bewegungsdaten;
Bestimmen (630), dass eine Umgebung der Vorrichtung eine erste Lichtbedingung aufweist;
als Reaktion auf das Bestimmen der ersten Lichtbedingung, Erfassen (640) eines ersten Frames der tragbaren Vorrichtung durch eine Kamera (112, 412, 522) unter Verwendung einer ersten Belichtungszeit;
Identifizieren (650), in dem ersten Frame, eines Musters von Lichtern, die auf der tragbaren Vorrichtung angeordnet sind;
Bestimmen, dass eine Umgebung der Vorrichtung eine zweite Lichtbedingung aufweist; als Reaktion auf das Bestimmen der zweiten Lichtbedingung, Erfassen (660) eines zweiten Frames der tragbaren Vorrichtung durch die Kamera (112, 412, 522) unter Verwendung einer zweiten Belichtungszeit, die länger als die erste Belichtungszeit ist;
Identifizieren (670), in dem zweiten Frame, von vorbestimmten Merkmalen der tragbaren Vorrichtung; und
Anpassen (680) der Pose der tragbaren Vorrichtung in einer Umgebung basierend auf (1) dem identifizierten Muster von Lichtern in dem ersten Frame und (2) den identifizierten vorbestimmten Merkmalen in dem zweiten Frame.

## Revendications

1. Un procédé (600, 601) comprenant, par un système informatique (800) :
la réception (610) de données de mouvement capturées par un ou plusieurs capteurs de mouvement d'un dispositif portable ;
la génération (620) d'une pose du dispositif portable sur la base des données de mouvement ;
la détermination (630) qu'un environnement du dispositif présente une première condition de lumière ;
en réponse à la détermination de la première condition de lumière, la capture (640) d'une première image individuelle du dispositif portable par une caméra (112, 412, 522) en utilisant une première durée d'exposition ;
l'identification (650), dans la première image individuelle, d'un motif de lumières disposées sur le dispositif portable ;
la détermination qu'un environnement du dispositif présente une deuxième condition de lumière ;
en réponse à la détermination de la deuxième condition de lumière, la capture (660) d'une deuxième image individuelle du dispositif portable par la caméra (112, 412, 522) en utilisant une deuxième durée d'exposition qui est plus longue que la première durée d'exposition ;
l'identification (670), dans la deuxième image individuelle, de caractéristiques prédéterminées du dispositif portable ; et
l'ajustement (680) de la pose du dispositif portable dans un environnement sur la base (1) du motif de lumières identifié dans la première image individuelle et (2) des caractéristiques prédéterminées identifiées dans la deuxième image individuelle.

2. Le procédé de la revendication 1, où la deuxième condition de lumière comprend une ou plusieurs conditions parmi :
un environnement présentant une lumière vive ;
un environnement présentant une source de lumière afin d'interférer avec le motif de lumières du dispositif portable ; et
le fait que la caméra (112, 412, 522) n'est pas apte à capturer le motif de lumières.

3. Le procédé de la revendication 1 ou de la revendication 2, où :
le dispositif portable est équipé d'une ou plusieurs unités de mesure inertielles UMI (122, 434) et d'une ou plusieurs diodes électroluminescentes, DEL, (240, 330, 432) infrarouges, IR ;
la première image individuelle est une image IR ; et
la deuxième image individuelle est une image en lumière visible.

4. Le procédé de l'une quelconque des revendications 1 à 3, où la pose du dispositif portable est générée à une fréquence plus rapide qu'une fréquence à laquelle la première image individuelle et la deuxième image individuelle sont capturées.

5. Le procédé de l'une quelconque des revendications précédentes, comprenant en outre :
la capture d'une troisième image individuelle du dispositif portable par la caméra (112, 412, 522) en utilisant la deuxième durée d'exposition ;
l'identification, dans la troisième image individuelle, d'une ou plusieurs caractéristiques correspondant aux caractéristiques prédéterminées du dispositif portable ;
la détermination de données de correspondance entre les caractéristiques prédéterminées et les une ou plusieurs caractéristiques ; et
le suivi du dispositif portable dans l'environnement sur la base des données de correspondance.

6. Le procédé de l'une quelconque des revendications précédentes, où le système informatique (800) comprend :
la caméra (112, 412, 522) configurée pour capturer la première image individuelle et la deuxième image individuelle du dispositif portable ;
une unité d'identification configurée pour identifier le motif de lumières et les caractéristiques prédéterminées du dispositif portable ; et
une unité filtrante configurée pour ajuster la pose du dispositif portable.

7. Le procédé de l'une quelconque des revendications précédentes,
où la caméra (112, 412, 522) est située au sein d'un dispositif monté sur la tête ; et
où le dispositif portable est un contrôleur séparé du dispositif monté sur la tête ; et de préférence où le dispositif monté sur la tête comprend un ou plusieurs processeurs, où les un ou plusieurs processeurs sont configurés pour mettre en œuvre la caméra (112, 412, 522), l'unité d'identification, et l'unité filtrante.

8. Un ou plusieurs supports de stockage non transitoires lisibles par ordinateur incorporant du logiciel qui est exploitable, lorsqu'il est exécuté, pour :
recevoir (610) des données de mouvement capturées par un ou plusieurs capteurs de mouvement d'un dispositif portable ;
générer (620) une pose du dispositif portable sur la base des données de mouvement ; déterminer (630) qu'un environnement du dispositif présente une première condition de lumière ;
en réponse à la détermination de la première condition de lumière, capturer (640) une première image individuelle du dispositif portable par une caméra (112, 412, 522) en utilisant une première durée d'exposition ;
identifier (650), dans la première image individuelle, un motif de lumières disposées sur le dispositif portable ;
déterminer qu'un environnement du dispositif présente une deuxième condition de lumière ;
en réponse à la détermination de la deuxième condition de lumière, capturer (660) une deuxième image individuelle du dispositif portable par la caméra (112, 412, 522) en utilisant une deuxième durée d'exposition qui est plus longue que la première durée d'exposition ;
identifier (670), dans la deuxième image individuelle, des caractéristiques prédéterminées du dispositif portable ; et
ajuster (680) la pose du dispositif portable dans un environnement sur la base (1) du motif de lumières identifié dans la première image individuelle et (2) des caractéristiques prédéterminées identifiées dans la deuxième image individuelle.

9. Le support de la revendication 8, où la deuxième condition de lumière comprend une ou plusieurs conditions parmi :
un environnement présentant une lumière vive ;
un environnement présentant une source de lumière afin d'interférer avec le motif de lumières du dispositif portable ; et
le fait que la caméra (112, 412, 522) n'est pas apte à capturer le motif de lumières.

10. Le support de la revendication 8 ou de la revendication 9, où :
le dispositif portable est équipé d'une ou plusieurs unités de mesure inertielles, UMI (122, 434) et d'une ou plusieurs diodes électroluminescentes, DEL, (240, 330, 432) infrarouges, IR ;
la première image individuelle est une image IR ; et
la deuxième image individuelle est une image en lumière visible ;

11. Le support de la revendication 8, de la revendication 9 ou de la revendication 10 où la pose de dispositif portable est générée à une fréquence plus rapide qu'une fréquence à laquelle la première image individuelle et la deuxième image individuelle sont capturées.

12. Le support de l'une quelconque des revendications 8 à 11, où le logiciel est en outre exploitable, lorsqu'il est exécuté, pour :
capturer une troisième image individuelle du dispositif portable par la caméra (112, 412, 522) en utilisant la deuxième durée d'exposition ;
identifier, dans la troisième image individuelle, une ou plusieurs caractéristiques correspondant aux caractéristiques prédéterminées du dispositif portable ;
déterminer des données de correspondance entre les caractéristiques prédéterminées et les une ou plusieurs caractéristiques ; et
suivre le dispositif portable dans l'environnement sur la base de la pose ajustée et des données de correspondance.

13. Le support de l'une quelconque des revendications 8 à 12,
où la caméra (112, 412, 522) est située au sein d'un dispositif monté sur la tête ; et
où le dispositif portable est un contrôleur distant séparé du dispositif monté sur la tête.

14. Un système comprenant : un ou plusieurs processeurs (802) ; et un ou plusieurs supports de stockage non transitoires lisibles par ordinateur (806) couplés à un ou plusieurs des processeurs et comprenant des instructions exploitables, lorsqu'elles sont exécutées par les un ou plusieurs des processeurs, pour amener le système à :
recevoir (610) des données de mouvement capturées par un ou plusieurs capteurs de mouvement d'un dispositif portable ;
générer (620) une pose du dispositif portable sur la base des données de mouvement ; déterminer (630) qu'un environnement du dispositif présente une première condition de lumière ;
en réponse à la détermination de la première condition de lumière, capturer (640) une première image individuelle du dispositif portable par une caméra (112, 412, 522) en utilisant une première durée d'exposition ;
identifier (650), dans la première image individuelle, un motif de lumières disposées sur le dispositif portable ;
déterminer qu'un environnement du dispositif présente une deuxième condition de lumière ;
en réponse à la détermination de la deuxième condition de lumière, capturer (660) une deuxième image individuelle du dispositif portable par la caméra (112, 412, 522) en utilisant une deuxième durée d'exposition qui est plus longue que la première durée d'exposition ;
identifier (670), dans la deuxième image individuelle, des caractéristiques prédéterminées du dispositif portable ; et
ajuster (680) la pose du dispositif portable dans un environnement sur la base (1) du motif de lumières identifié dans la première image individuelle et (2) des caractéristiques prédéterminées identifiées dans la deuxième image individuelle.
